# EUROPEAN PATENT SPECIFICATION

(11) **EP 1 414 979 B1**
(45) Date of publication and mention of the grant of the patent: **05.09.2007**
(21) Application number: 02749251.1
(22) Date of filing: 05.08.2002
(51) Int. Cl.: C12N 15/80, C12N 15/81, C12N 9/36

(54) **VECTOR FOR SITE-SPECIFIC INTEGRATION OF HETEROLOGOUS DNA SEQUENCES INTO THE RDNA SITES OF METHYLOTROPHIC YEASTS**
VEKTOREN ZUR ZIELGERICHTETEN INTEGRATION HETEROLOGER DNA SEQUENZEN IN DIE RDNA LOCI VON METHYLOTROPHEN HEFEN
VECTEUR POUR UNE INTEGRATION SITE-SPECIFIQUE DE SEQUENCES D'ADN HETEROLOGUES DANS LE SITE RDNA DE LEVURES METHYLOTROPHES

(30) Priority: 06.08.2001 IT MI20011728
(43) Date of publication of application: 06.05.2004
(73) Proprietor: BCS BIOTECH S.p.A., 09131 Cagliari (IT)
(72) Inventor: ROSSOLINI, Gian, Maria, I-53100 Siena (IT); RICCIO, Maria, Letizia, I-53100 Siena (IT); GALEOTTI, Cesira, I-53036 Poggibonsi (IT); POMPEI, Raffaello, I-09131 Cagliari (IT)
(74) Representative: Gervasi, Gemma
(86) International application number: PCT/IB2002/003086
(87) International publication number: WO 2003/014363

(56) References cited:
- WO-A-01/38510
- WO-A-99/57279
- ROSSOLINI G M ET AL: "KLUYVEROMYCES LACTIS RDNA AS A TARGET FOR MULTIPLE INTEGRATION BY HOMOLOGOUS RECOMBINATION" GENE, ELSEVIER BIOMEDICAL PRESS. AMSTERDAM, NL, vol. 119, no. 1, 1992, pages 75-81, XP000986022 ISSN: 0378-1119 cited in the application
- COX HELEN ET AL: "Constitutive expression of recombinant proteins in the methylotrophic yeast Hansenula polymorpha using the PMAI promoter." YEAST, vol. 16, no. 13, 2000, pages 1191-1203, XP002226641 ISSN: 0749-503X
- KONDO KEIJI ET AL: "A transformation system for the yeast Candida utilis: Use of a modified endogenous ribosomal protein gene as a drug-resistant marker and ribosomal DNA as an integration target for vector DNA." JOURNAL OF BACTERIOLOGY, vol. 177, no. 24, 1995, pages 7171-7177, XP002226642 ISSN: 0021-9193
- DATABASE EMBL [Online] EBI; 2 July 1986 (1986-07-02) LEDEBOER AM ET AL.: "Hansenula polymorhpa MOX gene for methanol oxidase" Database accession no. X02425 XP002226645 -& GÖDECKE S ET AL.: "Identification of sequences responsible for transcriptional regulation of strongly expressed methanol oxidase-encoding gene in Hansenula polymorpha." GENE, vol. 139, 1994, pages 35-42, XP002226643
- VASSILEVA ANA ET AL: "Expression of hepatitis B surface antigen in the methylotrophic yeast Pichia pastoris using the GAP promoter." JOURNAL OF BIOTECHNOLOGY, vol. 88, no. 1, 2001, pages 21-35, XP002226644 ISSN: 0168-1656

## Description

### Field of the invention

The technical field of the present invention concerns the vectors used for yeast transformation and production of recombinant heterologous proteins.

### Prior art

### Metilotrophic yeasts.

*Hansenula polymporpha* is a facultative metilotrophic yeast, i. e. which can also grow in media where methanol is the sole carbon and energy source.

Metilotrophic yeasts, a limited number of species belonging to the four *Hansenula, Pichia, Candida* and *Torulopsis* genera, were isolated in the early 70 following the great interest existing in that period to obtain biomass from methanol for use in animal feeding. Among all the isolated species, especially two taxonomically highly related, *H. polymorpha* and *P. pastoris,* were intensively studied for both the fermentation techniques and methanol metabolic biochemistry and physiology of methanol metabolism. Indeed, the two main aims of those studies consisted in obtaining potential business application and knowledge of the mechanisms involved in the biogenesis of important cellular organelles, the peroxisomes, which being ubiquitous in all eukaryotes from yeasts to man are however particularly abundant (and can be better studied) in cells growing in the presence of methanol (Gleeson MA and Sudbery PE, 1988, Yeasts, 4: 1-15).

Some methanol metabolic key enzymes, methanol- or alcohol-oxidase which converts methanol to formaldehyde and hydrogen peroxide (called MOX or AOX depending on whether one referres, respectively, to *H. polymorpha* or *P. pastoris),* dihydroxyacetone synthetase (DHAS) and catalase (CAT1), are located in peroxisomes. During the growth on methanol, the key enzymes of this metabolic pathway, in particular MOX, DHAS and a cytoplasmic enzyme, formate dehydrogenase (FMD), are produced at high levels and represents about one third of the total intracellular proteins (Sudbery PE, 1994, Yeast 10: 1707-1726).

Production of these enzymes is controlled at the transcriptional level and the promoters of the respective genes, isolated in yeasts *H. polymorpha* and *P. pastoris,* are strong and finely regulated (Gleeson MA and Sudbery PE, 1988).

The presence of extremely strong and also regulatable promoters, together with the ability to grow at high cellular densities (about 150 grams of dry cellular weight per litre of culture medium) using inexpensive substrates such as methanol and/or glycerol, stimulated, in the last 15 years, the study of metilotroph yeasts as an alternative system to *Saccharomyces cerevisiae* for large scale production of heterologous proteins (Gellissen G and Hollenberg CP, 1997, Gene 190: 87-97).

### Expression of heterologous proteins in metilotroph yeasts.

The expression systems obtained in *H. polymorpha* and *P. pastoris* utilize the *FMD* promoter and, more frequently, the *MOX* promoter (called *AOX* in *Pichia).* In both these organisms, these promoters are subjected to a strong induction by methanol and the repression by glucose.

In *P. pastoris* promoter activity is induced only by the presence of methanol and therefore the fermentation process requires two steps: growth on glucose and subsequent induction with methanol: growth on glucose indeed represses promoter activity so that the product to be expressed, synthesised at very high levels in the presence of methanol, does not interfere with the growth of the producer micoorganism, i. e. with the achievement of a good biomass level, but is expressed only after growth has occurred (when glucose in the culture medium is depleted and methanol is introduced) .

On the contrary, in *H. polymorpha* it is possible to obtain a *one-step fermentation* in the presence of glycerol or even a glycerol/methanol mixture, so as high yields are more rapidly attained: in *Hansenula,* indeed, growth on glycerol provokes a derepression of methanol-inducible promoters until obtaining not more than 20% of the maximum induction level, without therefore interfering with the producer microorganism growth (Hollenberg CP and Gellissen G, 1997, Current Opinion in Biotechnology 8: 554-560).

Yields attainable in *H. polymorpha* are, indeed, among the highest obtained with yeast-based systems; further, as this species secretes only a few proteins in the culture medium, the secreted heterologous product often represents the majority of extracellular proteins.

In a recent work (Mayer A F et al., Biotechnology and Bioengineering (1999) 63: 373-381), the authors describe the construction of *H. polymorpha* recombinant strains which are able to secrete exceptionally high phytase concentrations, which is active in the culture medium (13.5 g/L), where the heterologous enzyme represents more than 97% of the total accumulated proteins.

Another *H. polymorpha* advantageous feature, as par as the fermentation processes are concerned, is to be thermotolerant, i. e. it is able to growth properly in a temperature range between 30 and 42°C, with an *optimum* at 37°C, while both *P. pastoris* and *S. cerevisiae* have a growth *optimum* at 30°C (Sudbery PE, 1994).

Presently, *H. polymorpha* has been used mainly for industrial uses due to its favourable fermentation features, that is, in particular, thermotolerance and the one-step high yield fermentations, which do not require the unavoidable use of methanol. Many products made in *H. polymorpha* obtained the Food and Drug Administration's clearance and a hepatitis B vaccine produced in this yeast has already been introduced in the market (Hollenberg CP and Gellissen G., 1997). *Hansenula and Pichia expression vectors*

Until now, no endogenous plasmids have been isolated from *H. polymorpha* and *P. pastoris:* however, replicative vectors used in these two yeasts are maintained in an episomal state due to ARS sequences *(autonomous replication sequences)* derived from S. *cerevisiae* genomic sequences *(S. cerevisiae LEU2* and *URA3* genes for example have at different extents ARS activity in *Hansenula* and *Pichia)* or isolated from *Hansenula* and *Pichia* genomes.

Expression systems in these two yeasts (based on both "replicative" and "integrative" vectors) make use of leucine and uracile *(H. polymorpha),* or histidine auxotrophic mutants (both *H. polymorpha* and *P. pastoris),* as hosts; correspondingly, vectors used for transformation bear genes which complement respective mutations (ura, orithidine 5'-phosphate dehydogenase; *leu,* β-isopropyl malate dehydogenase; *his,* histidinole dehydogenase. In the majority of cases, plasmids constructed to transform *H. polymorpha* contain *S. cerevisiae LEU2* or URA3 genes: to this aim, Bogdanova and coll. reported (Bogdanova Al. et al., Yeast,1995 11: 343-353.) that a replicative-type vector containing the *H*. *polymorpha LEU2* gene *(HLEU2)* yields either very unstable transformants or alternatively stable transformants but heterogeneous because of the incorporation of different fragments of *Hansenula* genome.

Dominant selection systems were also prepared in *P. pastoris* and *H. polymorpha,* by inserting the amino glycoside 3'-phosphotransferase bacterial gene in the vector and selecting the transformants for resistance to G418 antibiotic (Hollenberg CP and Gellissen G, 1997).

The transformants containing plasmids able to replicate autonomously require a constant selective pressure and have been proved to be mitotically unstable for large scale production of heterologous proteins. The strains containing integrated copies of an expression cassettes resulted, on the other hand, generally more stable even in the absence of a selection for the plasmid marker. Furthermore, multiple copy integration of the expression cassette increases the heterologous product yield.

In *P. pastoris,* where, differently from *H. polymorpha,* two alleles of the *AOX* gene exist, integrative vectors for homologous integration into *AOX1* locus were prepared (either additional or insertional, or, as in case of the lnvitrogen system, substitutive or replacement); alternatively, some systems envisage integration into the *HIS4* locus. In both cases, integration into *P. pastoris* occurs frequently and is stable and generally takes place in single copy; however, substitution of the *AOX1* structural gene affords a strain which grows slowly on methanol.

Differently to what happens in *P. pastoris,* in *H. polymorpha* (Faber KN, et al., 1992, Journal of General Microbiology 138: 2405-2416) targeted integration attainable by homologous recombination into a target site, takes place with low frequency: integration frequencies not more than 1 to 22% have been obtained using the *MOX* gene or the amino oxidase gene and reaching an insertional-type integration which does not replace the target (Faber KN *et al.,* 1992). On the contrary, the frequent integration of replicative-type plasmids, thanks to a non-homologous recombination mechanism, represents a *H. polymorpha* specific feature, opposed to a homologous recombination low frequency. Integration which occurs by non-homologous recombination is not targeted, that is, it involves random sites in the genome: this is a drawback, because part of the integration events can take place into non-appropriate sites giving rise to not very viable or unstable transformants, with low yields expression. Further, being the transformants obtained according to random integration events, different in number, but especially in the localisation of the integrated cassettes, the selection of the best clones is difficult and their genetic analysis is rather complex, if not impossible.

More, it has been proven that in *H. polymorpha* the copy number of integrated plasmids is amplified (tandem amplifications at the integration locus) following some alternate growth cycles in selective and non-selective medium (Faber KN *et al.,* 1992; Gatzke R, et al., 1995, Applied Microbiology and Biotechnology 43: 844-849).

In *P. pastoris,* a multiple integration (3 to 18 copies) has been obtained including, in the expression construct, the gene encoding for a bacterial amino-glycoside 3'-phosphotransferase and selecting for high levels of G418 resistance (Scorer CA, et al., 1994, Bio/Technology 12: 181-184): also in this case, however, integration took place into random sites.

With a similar strategy, some authors have demonstrated that, in *H. polymorpha* too, the copy numbers of replicative-type plasmids integrated into the genome may depend on the plasmid marker used to select the transformants, and therefore the strength of the selective pressure. An example of the outcome variability of the integrative events into *H. polymorpha,* is the integration of the S. *cerevisiae* derived *URA3* gene, described in Gatzke R et al., 1995. *URA3* gene is expressed at low levels into *H. polymorpha* so that, when transforming Ura⁻ strains with a replicative vector which contains this marker, the selection process of Ura⁺ transformants favours cells with multiple copies (integrated into random sites) of the plasmid.

Other authors (Agaphonov MO et al. Yeast (1999) 15: 541-551) obtained a multiple integration (more than 100 copies) by non-homologous recombination in the following manner: they use a replicative vector (thanks to the presence of the HARS36 sequence) containing the *H. polymorpha LEU2* gene deleted in the promoter region *(HLEU2-d)* to transform a Leu strain; the receiving strain they use however carries a *HLEU2* structural gene almost completely disrupted, deleted, and in this way plasmid integration by homologous recombination (and in single copy) into *HLEU locus* is prevented. Using this technique, the authors obtain two kinds of transformants: 1) transformants able to grow slowly on a selective medium, containing a high number of copies integrated into the plasmid (100 to 120), yet inadequate, as the same authors observe, to restore the complete prototrophy in a strain bearing a disrupted *HLEU* gene; or, 2) transformants able to grow rapidly on a selective medium, but which are unstable and which shortly originate populations with a slow growth rate.

The reasons why this instability occurs are not clear: in some cases it has been ascribed to the integration *locus,* in other cases to the type or dimensions of the integrated sequences. Different hypothesis have been proposed, such as that repetitions (repeats) of integrated copies beyond 100 units are unstable, as Lopes and coll. propose (Lopes TS et al.: Yeast (1996) 12: 467-477) in a work wherein multiple plasmid integration into the *S. cerevisiae* rDNA (ribosomal DNA) *locus* is described. In the vectors described in this case however, exogenous DNA is flanked only at one end by yeast rDNA sequences (whose length is up to several kilobases), which direct Integration of the whole plasmid into the chromosome in a position adjacent to the target (with an insertional-type, and not substitutive, integration which is added to the target and does not replace it). With these vectors Lopes and coll. obtain integrants with a high copy number (up. to 100-200 integrated copies) some of which are unstable. One of the hypotheses formulated by the these authors is that insertional integration in high copy number of large plasmids (up to 12 Kb) increases the chromosomal *locus* size to such an extent to render it unstable; the same Integration in high copy number of bacterial sequences contained in the plasmid totally Integrated in this kind of event, may represent a further instability reason.

Rossolini G.M. et al. (Gene, 1992, 119 : 75-81) also disclose the use of the ribosomal *locus*, namely in *Kluyveromyces lactis,* a non-methylotrophic yeast, to attain site-specific integration. Rossolini et al. have analyzed targeting of the 25S rDNA gene to the chromosomal rDNA cluster of *K. lactis* by using a replacement vector, and obtained *K. lactis* transformants carrying multiple copies of the replacement cassette inserted into the rDNA chromosomal locus.

W00138510 discloses an integration vector for the targeted and stable integration of heterologous DNA in the genome of an host organism, for example a methylotrophic yeast such as *Hansenula polymorpha,* so as to enable the latter to produce recombinant proteins. The provided integration vector comprises a rDNA sequence from a yeast or a mycete, a non-deficient selection marker gene and an expression cassette. The latter comprises a promoter element, an area of heterologous or genuine gene coding for a desired protein and a terminator element, all of which are functional in the host organism.

Cox H. et al. (Yeast, 2000, 16: 1191-1203) disclose the use, in *Hansenula polymorpha,* of the constitutive promoter PMA1 for programming the expression of two heterologous proteins, glucose oxidase and recombinant human serum albumin (rHA). According to Cox H. et al, the constitutive promoter provides an useful additional facility to the *H.polymorpha* expression system because it allows a simplified fermentation regime and enables use of methanol to be avoided.

In *H. polymorpha,* In which site-specific integration events generally occur at a low efficiency, Sohn and coll. (Sohn J-N et al.: Applied Microbiology and Biotechnology, 1999, 51: 800-807) presented data relative to an Integration system in which the number of integrated copies can be controlled and the procedure to select multiple Integrals be simplified, compared to previous systems. Indeed, Sohn and coll. use a vector containing a telomeric *HARS* and the gene of a bacterial amino glycoside 3'-phosphotransferase *(APH)* under the control of a *H*. *polymorpha* promoter (glyceraldehyde-3-phosphate dehydrogenase) deleted in different positions. The presence of a selection marker under the control of a defective promoter implies a reduced *APH* expression, and therefore permits to rapidly select multiple integrants, with a copy number up to 50, in a fashion related to the G418 increasing concentration used in the selective medium. In this system, integration occurs at the terminal regions of *Hansenula* chromosomes, following homologous recombination between the vector *HARSs* and the genomic *HARSs* (this telomeric *HARS* actually promotes this kind of integration). However, the same authors observe that the telomers represent regions that are essential for chromosome stability and replication, so that a multicopy plasmid integration into these regions could easily determine instability both of the chromosome and of the introduced gene.

A similar approach has been employed in the system developed in *H. polymorpha* to express hepatitis B virus L and S antigens by Janowicz ZA et al. (Yeast, 1991, 7:431-443). In this case, however, the authors do not use a system capable to control the number of integrated copies, but select the transformants containing the optimal number of integrated copies of the L and S antigen genes.

In conclusion, in metilotrophic yeasts, and in particular in *H. polymorpha* and *P*. *pastoris,* the frequency with which multiple integrants are obtained in high copy number by site-specific integration, is rather low and poorly predictable: for this reason, the procedures set up until now to obtain multiple integrants have so far required fairly elaborate methods because of the need of selecting and analysing a very high number of transformants, as well as because of the need of their optimisation by growth cycles in selective and non-selective media.

### Description of the figures

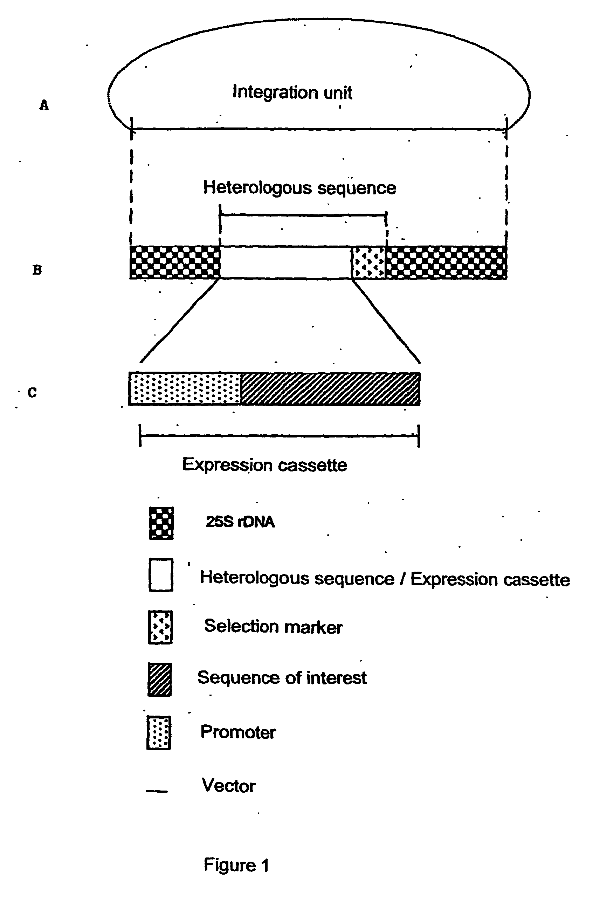

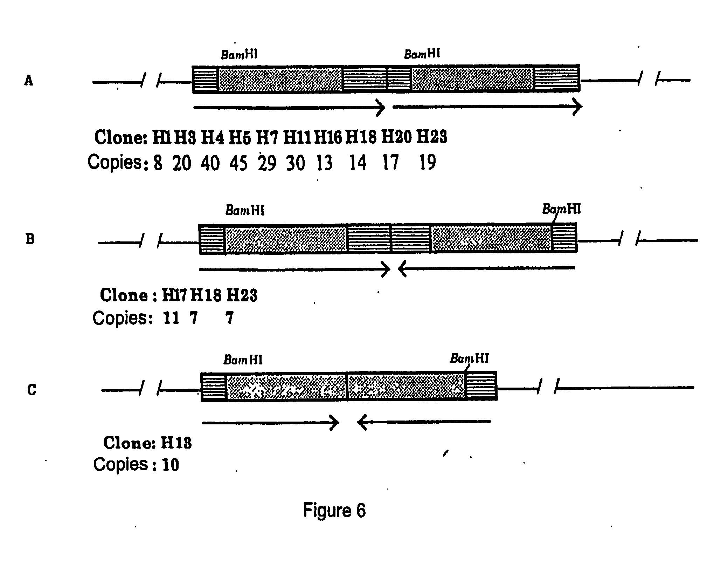

Figure 1: Schematic representation of the integration unit and of the vector according to the invention.
   The figure shows the vector A) containing the integration unit B), composed of a heterologous DNA sequence, C), comprised within two integration boxes consisting of the 25S ribosomal RNA coding sequences. According to a preferred embodiment of the invention, the heterologous DNA sequence consists of an expression cassette. The selection marker is always included in the heterologous DNA sequence.

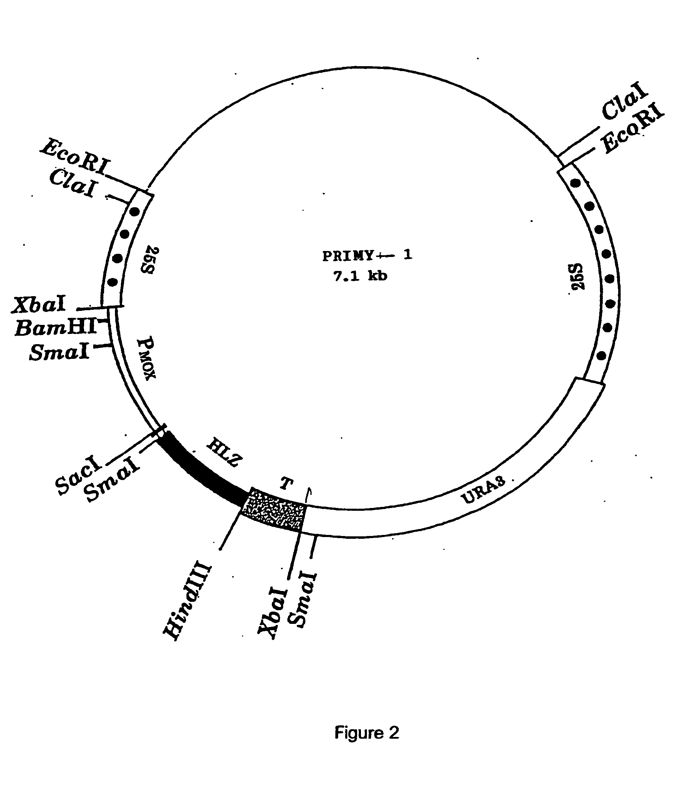

Figure 2: Restriction map of pRIMY-1 plasmid vector.
   The figure shows the main restriction sites utilised in the vector construction. The black dotted zones represent the S. *cerevisiae* 25S rDNA (25S) sequences. The *Xba*I*-Xba*I segment which can be seen in the figure spans 1.9 kb and contains the expression cassette formed by the MOX promoter (pMOX, *Xba*I*-Sac*I segment) and the human lysozyme cDNA (HLZ), preceded by the *K. lactis* killer toxin signal sequence (Sacl-Xbal) and followed by the transcription terminator (T) derived from 2 µm plasmid (Sacl-Xbal segment). As illustrated in the figure, cutting with *Cla*I releases the integration cassette as a 4.7 kb Clal-Cial fragment.

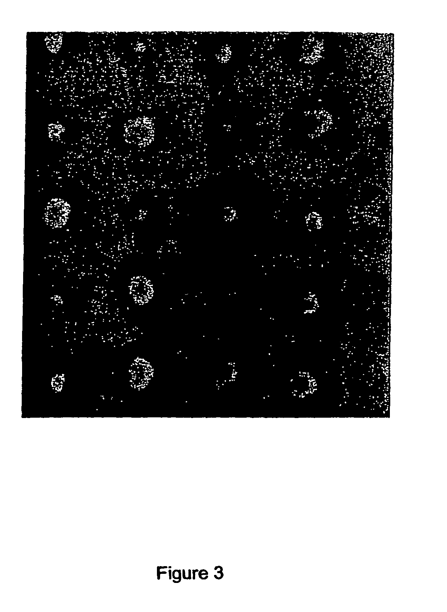

Figure 3: Qualitative analysis of *H. polymorpha* transformants for their ability to produce human lysozyme.
   The assay, called *lysoplate assay,* was performed on agar YPM medium plates in which a 1:100 diluted suspension of *Microccocus luteus* cells as a substrate of the lytic activity has been included (the stock solution of *M*. *luteus* cells is prepared in 70 mM phosphate buffer pH 6.3 and the cells, resuspended until obtaining an O.D.₆₀₀ of 70-80, are killed by two autoclave cycles). Rings of cellular lysis are evident around the colonies transformed by the vectors of the invention, showing the presence of lysozyme bacteriolytic activity.

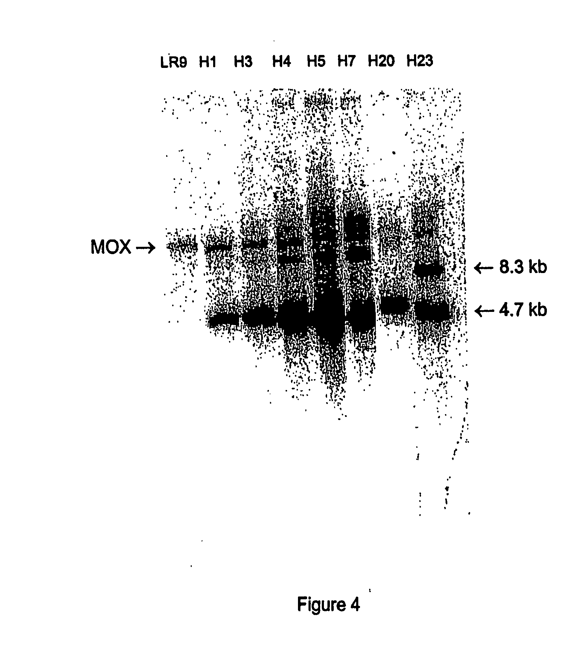

Figure 4: Copy number analysis of the integration units integrated in *H*. *polymorpha* according to the invention.
   Same amounts (2 µg) of total DNA extracted from LR9 strain (receiving strain, used as a control) and from some transformants were digested with *Bam*HI and subjected to agarose gel electrophoresis (0.8% agarose). Following the electrophoretic run, the gel was transferred onto nitrocellulose and hybridized with a probe formed by the *MOX* promoter. The hybridization result, reported in the figure, was then subjected to densitometric analysis to define the number of integrated copies into every transformant clone (the LR9 control strain band being considered equivalent to one unit). Compare to the summarising scheme in figure 6.

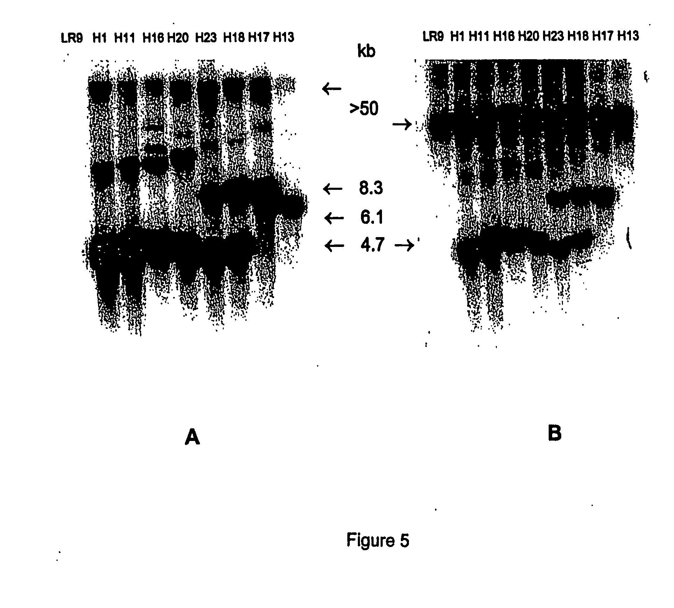

Figure 5: Distribution analysis of the integration sites in some transformants.
   The distribution of the Integration sites was analysed in transformants H1, H₁₁, H13, H17, H18, H20 e H23: LR9 strain was inserted as a control. Total DNA samples, digested with *Bam*HI, were resolved on agarose gel and transferred onto nitrocellulose. The hybridization signal obtained with a probe which consists of the human lysozyme cDNA is shown in A and the signal obtained with a probe which consists of the S. *cerevisiae* 25S rDNA *Eco*RI-*Bgl*II fragment is shown in B.
Figure 6: Schematic reconstruction of distribution of the Integration sites in different transformants.

In this figure, the results obtained in figures 4 and 5 were schematically interpreted and represented. Arrows indicate the length and orientation of the integrated units; grey rectangles indicate the expression cassette for human lysozyme plus *URA3* gene, while dotted rectangles, with 2 different lengths, show the two 0.55 and 1.1 kb *"integration boxes".* Comparative analysis of the intensities of the bands of the transformants, compared to that present In LR9 (one pMOX copy), conducted by a densitometer, allowed the quantification of the Integration unit copy number present in every clone. A) B) e C) represent 3 different integration events: A) head-to-tail orientation; B) head-to-head orientation; C) head-to-tail orientation with deletion of an integration box..

### Summary

The present invention refers to a vector for site-specific integration of heterologous DNA sequences into metilotrophic yeast strains. By using such vector, a heterologous DNA sequence Is Integrated at a high frequency into multiple sites as far as the integration target is constituted by repeated and multiple copy units throughout the target genome.

In a preferred embodiment, the vector is a plasmid vector for multicopy site-specific integration of an integration unit comprising an expression cassette into metilotrophic yeasts.

The expression cassette comprises a promoter active in metilotrophic yeasts, a heterologous DNA sequence, which codes for a protein, polypeptide or peptide of interest and the transcription of which is regulated by the aforesaid promoter, transcription terminating sequences and a selection marker that is preferably chosen among *LEU2, URA3, HIS3* and *G418R* genes. The expression cassette is flanked upstream (5') and downstream (3') by at least 50 bp of the sequence coding for the yeast *S.cerevisiae* 25S ribosomal DNA (Genbank access No.J01355).

The invention also refers to an integration unit for metilotrophic yeasts comprising an expression cassette comprising a promoter active in metilotrophic yeasts, a heterologous DNA sequence, which codes for a protein, polypeptide or peptide of interest and the transcription of which is regulated by the aforesaid promoter, transcription terminating sequences and a selection marker that is preferably chosen among *LEU3, URA3, HIS3* and *G418R* genes. The expression cassette is flanked upstream (5') and downstream (3') by DNA sequence coding for the yeast *S. cerevisiae* 25S ribosomal DNA (Genbank access No.J01355), or by fragments of such sequence that are at least 50 bp long.

The invention further refers to the microorganisms transformed with the vectors or the integration units as they are defined, and the pRIMY-1 vector deposited at the CNMC collection (Collection Nationale de Cultures de Microorganismes, Institut Pasteur) on 18^{th} July 2001, number I-2705.

A process for recombinant protein expression and a process for integration of heterologous DNA sequences into metilotrophic yeasts, those of the *Hansenula* genus being particularly preferred, are further aspect of the invention.

### Detailed description of the invention

A vector for site-specific integration of heterologous. DNA sequences into metilotrophic yeast strains is the object of the present invention.

Metilotrophic yeasts are particularly advantageous for fermentative applications because they can grow using methanol or other simple carbon sources as a sole carbon source.

The yeast *Pichia, Hansenula, Candida,* and *Torulopsis* genera belong to metilotrophic yeasts. The *Hansenula* genus is particularly preferred to the embodiment of the present invention, in particular *H. polymorpha.* In this yeast, the activity of the promoters generally used to produce heterologous proteins, for example the MOX, FMD, DHAS promoters (Faber KN et al., 1995, Yeasts*)* is repressed by glucose strongly induced by the presence of methanol, and partially induced (derepressed) by the presence of glycerol or a glycerol/methahol mixture, or also glucose limiting concentrations. These features allow to carry out a one-step fermentation in *H. polymorpha* in the presence of simple and inexpensive carbon sources, for example glycerol (or methanol), obtaining high product yields more rapidly than what required for the same induction level in other yeasts, such as *P. pastoris,* which on the contrary requires a two-step fermentation.

Furthermore, since *Hansenula* naturally secretes only a few proteins in the extracellular space, the heterologous ones directed therein are generally the most abundant and easy to purify.

Another feature of this yeast genus, which is extremely advantageous in fermentative processes, is its thermotolerance. Actually, *H. polymorpha* grows well in a temperature range between 30 and 42°C, with an *optimum* at 37°C, while both *P. pastoris* and *S. cerevisiae* grow well mainly around 30°C.

The authors of the present Invention surprisingly found that when the integration vector for metilotrophic yeasts, represents the main object of the present invention, and which contains ribosomal sequences flanking upstream and downstream a heterologous DNA sequence (a "integration unit"), is used to transform yeast, such heterologous sequence is integrated at a high frequency and into multiple sites in the chromosome, as the ribosomal *locus* and consequently the integration target site is formed by repeated units.

The multiple integration event is followed by duplication events of the integrated sequence following growth cycles in selective and non selective medium which further increase the Integrated copy number. Consequently, such integration is further defined as a multicopy integration.

To the aims of the present invention, by heterologous DNA sequence is meant any DNA sequence which is intended to be integrated in multiple copies and at a high frequency into the yeast genome, and which is preferably derived from organisms different from yeast (heterologous sequence), but which also derives from the yeast itself (homologous). The heterologous sequence, when flanked upstream (5') and downstream (3') by sequences (also called *integration boxes)* coding for ribosomal RNA or its fragments thereof and, moreover comprising an opportunely regulated selection marker for the expression in yeast In such a way to permit the selection of the transformed clones, represents an integration unit.

In a preferred embodiment, the integration unit comprises an expression cassette for a heterologous gene from which the corresponding recombinant protein is intended to be obtained and the selection marker.

A scheme of one embodiment of the Integration unit and integration vector disclosed in the present invention is presented in figure 1.

The integration unit according to the invention, included the heterologous DNA sequence, is stably integrated In multiple sites and in multiple copies into the yeast genome, together with the ribosomal flanking sequences which replace the target sequences in the ribosomal *locus,* when it is used according to techniques known to transform the yeast host. '

Thus, this kind of integration is defined as substitutive, alternatively to insertional integration wherein the heterologous sequence consisting of the entire vector, is added alongside the target site. Substitutive integration is advantageous as compared to insertional because the heterologous DNA which is integrated into yeast genome is only the one comprised within the ribosomal DNA sequences, so that plasmid sequences of bacterial origin, which are useless and harmful as well for the yeast and which may be present when the integration unit is cloned into a plasmid vector (replication origin and resistance genes) are excluded from integration.

In a preferred embodiment the integration unit, which is a linear DNA molecule, is cloned into a plasmid vector containing all the genetic elements useful to allow its amplification in bacteria, such a replication origin. Such a vector is circular DNA.

According to the present invention, the vector allows a targeted site-specific integration into the ribosomal *locus*, composed of the multicistronic rDNA of metilotroph yeasts, preferably of *H. polymorpha.*

*H. polymorpha* carries about 25 repeated units of rDNA genes, which as in *Saccharomyces cerevisiae* are organised in groups or *"clusters"* on the same chromosome. Despite the fact that the rDNA *cluster* in *H*. *polymorpha* is represented by a lower unit number compared to S. *cerevisiae* (25 in comparison with about 100 for S. *cerevisiae),* the integration frequency of the integration cassette into the target site is notably increased with regard to a target present only in single or double copy in the yeast genome, such as for example MOX or AOX *loci,* and the integration unit, integrating into different of the ribosomal *locus*, undergoes a first "amplification".

Afterwards, the transformants containing the integrative unit in stable form are grown alternating minimal media and complete media, according to procedures and growth media (selective or not selective) known to the person skilled in the art, and the integrative unit is further amplified by subsequent tandem duplications in every integration site, giving rise to stable multicopies.

In *H. polymorpha* each of the 25 units is 8.1 kb long and codes for 18S, 5.8S, 25S e 5S RNAs.

Surprisingly, the authors of the present invention have observed that non-contiguous segments of these DNA genes, derived from yeasts of different species and whose minimal length is 50 bp when inserted downstream and upstream a heterologous DNA sequence, are sufficient to assure homologous recombination of such a sequence in the target ribosomal *locus* in metilotrophic yeasts. In particular, in *Hansenula* genus, a stable and at a high frequency site-specific recombination event is particularly surprising.

25S RNA coding sequences are particularly preferred as integration target sites and therefore as sequences (integration boxes) flanking the heterologous DNA sequence or the expression cassette on the vector. The ribosomal *locus* is highly conserved in yeast: for example, about 90% homology exists between the ribosomal sequences coding for *Saccharomyces cerevisiae* and *Hansenula polymorpha* 25S RNAs, which are yet taxonomically rather distant. The high degree of homology among these yeasts sequences provides integration by homologous recombination of the vectors or the integration cassettes according to the present invention to occur into all yeasts studied so far. Thus the vector or the integration unit of the invention are used in all the yeasts, also if not metilotrophic, provided that the specific regulatory sequences, such as for example promoters, are replaced with sequences suitable for the expression in the particular transformed yeast host.

However, the high frequency of the integrative events into the target sites and the subsequent duplication (amplification) of the cassettes there integrating are particulary advantageous in particular in **the** *Hansenula* genus yeasts making this host particularly preferred for the application of the invention.

Indeed, the high integration frequency due to the high number of target sites present in the ribosomal *locus,* in addition to the duplication event (tandem amplification) of heterologous DNA copies obtained by growing the transformant clones alternating rich and minimal medium, on one hand increases the number of integrated copies and on the other reduces the procedures downstream the transformation, which are instead necessary in the known art vectors, to select multicopies clones.

Furthermore, the integration event into different target sites of the ribosomal locus increases the stability of the transformant clones and thus represents a further particularly advantageous aspect of the present invention.

A high integration frequency, such as that obtained with the vectors of the present invention, moreover represents an at all unexpected event for *Hansenula:* actually, as widely documented in literature, the integrative events into this yeast are generally rare and occur at a low frequency. Indeed, it is known that multicopy integration into *Hansenula* is usually associated to random integrative events (non site-specific), opposite to what happens in other non-metilotrophic yeasts.

The integration event mediated by the ribosomal sequences utilised in the vector according to the present invention is used for any heterologous and not heterologous DNA sequence type. Therefore, are comprised in the present definition of heterologous DNA sequence both expression cassettes defined as DNA sequences comprising a gene or heterologous cDNA from which the production of the corresponding recombinant protein is intended to be obtained and which are therefore opportunely regulated by sequences specific for the metilotrophic yeast (such as for example promoters), as well as any heterologous yeast DNA sequence that is usefully integrated in multiple copies into the yeast genome even if not necessarily coding for a protein.

The selection marker present in the heterologous DNA sequence is chosen preferably among *LEU2, URA3* and *HIS3* genes, which allow to recover the prototrophy phenotype in auxotrophic yeast strains for leucine, uracile or histidine, respectively. Other genes or selection markers can be used in the vector or integrative unit according to the invention, such as dominant selection markers, for example that conferring resistance to G-418 antibiotic (also indicated with G-418^{R} or bacterial amino glycoside 3'-phosphotransferase, *APH)* or encoding for pleomycine resistance (Hollenberg CP and Gellissen G, 1997). The selection procedure of transformants depends on the chosen selection marker, according to the methods known to the person skilled in the art. In case the organism is transformed with a selection marker which confers resistance to a particular antibiotic, the selective medium is composed of the medium containing the antibiotic against which resistance has been conferred, for example G-418 containing medium or another, according to known methods. In the preferred embodiment and according to the embodiment wherein the marker is selected among *LEU2* or *URA3* or *HIS3* genes, selection is performed on minimal medium which does not contain the component against which, by means of transformation, prototrophy is intended to be restored (uracile or leucine or histidine) according to the methods known in the art. Thus, in this type of application the minimal medium represents the "selective medium". *URA*3 and *LEU*2 selection markers are particularly preferred. Also the selection marker is regulated by appropriate transcription promoter and terminator, which are able to regulate its transcription in the selected yeast genus.

The integration unit comprises an expression cassette, which comprises the following functional regions: a) a promoter active in metilotrophic yeasts which regulates the transcription of the heterologous sequence in an inducible or in a constitutive fashion; b) a heterologous DNA sequence coding for the protein, polypeptide or peptide of interest; c) appropriate transcription termination sequences, such as for example the transcription terminator derived from S. *cerevisiae* 2µm plasmid FLP gene; d) a selection marker, preferably chosen among *LEU3, URA3, HIS3, G418R*. *MOX* and *FMD* promoters are, in a particularly preferred embodiment of the invention, the promoters which regulate the heterologous gene or DNA sequences expression, because they are induced to transcription by methanol in a particularly efficient way. However, other promoters specific for metilotrophic yeast are useful, such as for example CAT1 (catalase-1) and *DHAS* (dihydroxyacetone synthetase) (Gleeson MA and Sudbery PE, 1988), whose induction levels by methanol are comparable to *MOX* and *FMD.* Preferably, the promoter is the *H*. *polymorpha* methanol oxidase promoter, corresponding to Seq IDN 3.

The heterologous protein or peptide or oligopeptide is preferably expressed in secreted form, by cloning a signal sequence which is preferably of yeast, at the 5' of the DNA sequence coding for the protein of interest. Such signal sequences (leader peptides) are preferably selected among: that, of *K*. *Lactis* and S. *cerevisiae* killer toxin, that of the *S. cerevisiae* MFα1 (Mating Factor-1) factor *leader* sequence, that of the *Aspergillus niger* glucoamylase and the pre-pro-sequence derived from the *Schwanniomyces occidentalis* glucoamylase-1 gene, *GAM1* (Gellissen G and Hollenberg CP, 1997; Hollenberg CP and Gellissen G; 1997). The *K*. *Lactis* killer toxin signal sequence (access number: X00762) is particularly preferred.

Integration of the integration unit into the target site preferably occurs by transformation with the expression cassette alone, cut from the whole vector by using appropriate restriction enzymes, selected in such a way not to disrupt the integrative unit. In its preferred embodiment, which is the pRIMY-1 vector deposited at the CNMC collection (Collection Nationale de Cultures de Microorganismes, Institut Pasteur) on July 18th 2001, number I-2705, the integrative unit is extracted by restriction with Clal enzyme, that cuts each side of the integration boxes. The integration cassette can optionally be purified after the enzymatic restriction according to the methods known in the art, for example by electrophoretic separation on agarose gel. Alternatively, the vector can also be used as such without enzymatic digestion or after a digestion which linearizes in one position, provided it is external to the integration unit.

According to the vector of the invention, rDNA fragments *(integration boxes)* at the ends of the integration unit are composed of sequences derived from the *locus* coding for *Saccharomyces cerevisiae* rDNA. Such *locus* contains the DNA coding for 25S, 18S, 5.8S and 5S RNAs, which can all be equally used as "integration boxes". Non-contiguous segments of these genes with a minimal length of 50 bp, inserted downstream and upstream of a heterologous DNA sequence are sufficient to assure homologous recombination to such heterologous sequence in the target ribosomal *locus.* The sequences derived from the gene coding for 25S RNA are particularly preferred, even more preferred are those comprising the 1-548 EcoRl-Bc/l fragment (0,55 kb) corresponding to Seq IDN 1 and the 1545-2651 *Bgl*II*-Eco*RI fragment corresponding to Seq IDN 2 (1.1 kb), which belong to the sequence identified with GenBank access number J01355 and, which are located at 5' and 3', respectively, of the heterologous DNA sequence (see Fig. 2). Adjacent fragments or fragments comprising the sequences identified by them or different from them, but anyway derived from the genes coding for *S. cerevisiae* 25S, 18S, 5.8S and 5S RNAs or of other yeasts can in any case be used in the present invention.

Apart from the preferred embodiment, other sequences having analogous functions (transcription terminators, promoters, sequences coding for signal peptides, selection genes) and derived both from yeasts and other organisms, can be used in the expression cassette to ensure the expression of the gene of interest or selection marker, according to what is known in the state of the art. For example, integration units can be used that comprise one or more expression cassettes generally consisting of: a sequence coding for a yeast ribosomal RNA or its fragments, inserted according to the methods known in the art, upstream and downstream of an expression cassette for the gene, cDNA, or DNA fragment which is intended to be expressed in recombinant form, and carrying all the regulatory sequences necessary for expression, such as: promoter, *ribosome-binding-site,* optionally a signal sequence fused in the same reading *frame* of the gene of interest, transcription terminator and others known to the man skilled' in the art,

Plasmid vectors which can be amplified in bacteria, and comprising the integration unit according to the description, independently from the gene of interest cloned therein and the sequences apart from the integration unit useful for example for amplification in bacteria, are comprised within the scope of the present invention.

In pRIMY-1 vector, deposited at the CNMC collection (Collection Nationale de Cultures de Microorganismes, Institut Pasteur) on July 18th 2001, number I-2705, the gene of interest is the sequence coding for human lysozyme (data-bank accession number: M19045, 68-464 fragment), fused *in frame* with the *K. Lactis* toxin signal peptide sequence. In this way, the recombinant lysozyme is secreted outside the cells, where it can be easily detected and measured with a bacteriolytic activity assay on plates or "lysoplate assay" (Castañón MJ, et al., 1988, Gene 66: 223-234). The human recombinant lysozyme, cloned in this vector and secreted in *H. polymorpha,* is therefore only a reporter gene utilised just for the easiness of detection of the final product. It is clear however that the human lysozyme produced represents only an example of particular embodiment among the many existing. Hormones, enzymes, peptides, pharmacologically active substances, vaccines, or generally all the proteins efficiently expressed in yeast are examples of recombinant proteins produced by the use of the vector according to the present invention and which are therefore comprised In the scope of the invention.

Moreover the invention comprises vectors which can be amplified in bacteria comprising the amplification unit for metilotrophic yeasts as defined, independently from the cloned gene of interest and the sequences external to the integration cassette which are necessary to amplify the vector in bacteria, generically comprising at least: an *E.coli* replication origin, an *E.coli* resistance gene and a series of unique cloning sites, *polylinker* or *multicloning sites.*

According to a further embodiment of the invention, this further includes the microorganisms transformed with the previously described vectors, in particular bacteria such *E. coli,* a preferred embodiment of which is the strain transformed with the pRIMY-1 vector deposited at the CNMC collection (Collection Nationale de Cultures de Microorganismes, Institut Pasteur) on July 18th 2001, number I-2705, where the vector is maintained in order to carry out the cloning procedures of the sequences of interest, and the yeasts, preferably metilotrophic yeasts such as the *Pichia, Hansenula, Candida* and *Torulopsis* genera, transformed with the vectors or the integration unit of the present invention according to the methods known to the man skilled in the art. Metilotrophic yeasts of *Hansenula* genus, in particular *H. polymorpha,* are particularly preferred, among yeasts when transformed with the vectors or the integration unit of the present invention or with the pRIMY-1 vector, deposited at the CNMC collection (Collection Nationale de Cultures de Microorganismes, Institut Pasteur) on July 18th 2001, number 1-2705.

The vectors according to the invention or the purified integration unit, are taken up by yeasts or bacteria by means of transformation carried out according to the techniques known in the state of the art. According to a preferred embodiment of the present invention, yeast transformation occurs by the method described in

Faber et al. Journal of General Microbiology (1992) 138: 2405-2416).

A further object of the present invention is a method for homologous (site-specific), substitutive and multicopy integration of such sequences into the ribosomal *locus* of: *Pichia, Hansenula, Candida,* and *Torulopsis* metilotroph yeasts. Such integration is stable for more than 50-70 generations, and takes place with a high frequency.

The process according to the invention permits to integrate the integrative unit at the level of the DNA sequences coding for 25S or 18S or 5.8S or 5S RNAs, preferably 25S and is characterised by the use of the vectors according to the invention, containing downstream and upstream the integration unit the DNA sequences coding for such rRNAs.

In particular, when the vector of the present invention is used to transform *H. polymorpha* with a opportunely regulated gene or DNA sequence or cDNA, the transformant strains can be fermented by one-step fermentation, utilising simple and inexpensive carbon sources, such as for example glycerol and methanol, obtaining the product at high yields more rapidly than required for the same expression level in another yeast such as *P. pastoris,* which instead requires a two-step fermentation.

Since in a preferred embodiment of the invention the integration unit includes at least an expression cassette, defined as above and containing the gene of interest, the invention further relates to a process for the expression of recombinant proteins in metilotrophic yeasts, preferably of the *Hansenula* genus, more preferably *H. polymorpha,* characterised in that it uses the vectors or integration unit or yeast strains transformed with such DNA sequences according to the different embodiments described in the invention.

Such process essentially comprises the following steps: transformation of the yeast strains with the vectors or the integrative unit of the invention, selection of the transformants on selective medium, amplification of the integrative unit by alternating the growth on selective and non-selective media, selection of the best strains for productivity, one-step fermentation in culture media known in the art and comprising a simple carbon source such as glycerol or a glycerol/methanol mixture, or methanol and recovery of the exhausted culture broth or of the yeasts depending on whether the protein is secreted or not to purify the recombinant product. The selection step of the overproducing clones can be performed either by little scale fermentations or by genetic analysis to verify the copy numbers of the units which have been integrated into the genome. The fermentation step is conveniently carried out at temperatures between 30°C and 42°C, preferably between 34°C and 39°C, or more preferably between 36.5°C and 37,5°C. More in particular, the process for the expression of heterologous recombinant proteins includes, besides the yeast transformation process described by Faber et al. and briefly mentioned, the following steps:
- preparation of transformation-competent cells,
- transformation with the linearized vector or the integration unit, in case in the presence of carrier DNA,
- plating on selective medium and incubation of the transformed yeasts at a temperature between 28°C and 32°C, preferably 30°C for at least 48 hours, preferably 72 hours,
- alternate growth on selective and non-selective media, at a temperature between 28°C and 42°C, even more preferably between 36,5°C and 37,5°C,
- phenotypic analysis of the transformants with the best productivity features,
- one-step fermentation of the best strains on simple carbon sources.

The recovery step of the recombinant product can alternatively contemplate the recovery of the yeasts, in case the protein is produced intracellularly by the yeast, or the supernatant in case the protein is secreted.

According to a further aspect, the invention consists of a kit for the expression of heterologous proteins comprising the vector DNA containing the integration unit, preferably the pRIMY-1 DNA, which may be pre-digested with restriction enzymes allowing the cloning of the expression cassette of interest by replacing the one containing the lysozyme gene, and in case a yeast strain, preferably *H*. *polymorpha* optionally in a lyophilised form. Optionally the kit includes appropriate restriction enzymes.

### EXPERIMENTAL PART

### Example. 1: Construction of the pRIMY-1 vector.

The vector or integrative unit integration target according to the invention is the *H*. *polymorpha* multicistronic rDNA: in this yeast 25 repeated units of the rDNA genes have been identified which, as in *Saccharomyces cerevisiae,* are organised in *cluster* on the same chromosome.

The integration pRIMY-1 vector, deposited at the CNMC collection (Collection Nationale de Cultures de Microorganismes, Institut Pasteur) on July 18th 2001, number I-2705, is a substitutive-type vector *(replacement vector)* and contains an expression cassette and a selection marker cloned within DNA segments capable of recombinating with *Hansenula* chromosome in the rDNA *locus:* the homologous annealing on both ends of the target chromosome (double *crossing over)* causes the integrated exogenous DNA to replace the chromosomal segment which serves as an integration mark or *target.*

In *H. polymorpha,* every rDNA unit is 8.1 kb long and comprises the sequences coding for 18S, 5.8S, 25S and 5S ribosomal RNAs, whose sequence is published in Blandin G, et al., FEBS Letters (2000) 487: 76-81. In the inventive vector, the *integration boxes* (the recombination sequences) are two non-contiguous 0.55 and 1.1 kb respectively long fragments of the S. *cerevisiae* 25S rDNA corresponding to the 1-548 *Eco*RI*-Bcl*I fragment, 0.55 kb long and 1545-2651 *Bgl*II*-Eco*RI fragment, 1,1 kb long and belonging to the sequence identified with the accession number J01355 (GenBank).

In Figure 2 the pRIMY-1 vector map is illustrated. The integration unit (i. e. the part of the vector which integrates) contains an expression cassette and a selection gene. The expression cassette contains: the DL1 strain *H. polymorpha MOX* promoter, (PMOX in Figure 2; ref. Ledeboer AM, Edens L, Maat J, Visser BJW, Verrips CT, Eckart M, Roggenkamp R and Hollenberg CP: Nucleic Acids Research (1985) 13: 3063-3082), the cDNA sequence coding for the mature form of human lysozyme fused *in frame* with the *K. lactis* killer toxin signal sequence (HLZ) and the S. *cerevisiae FLP* transcription terminator (T) (HLZ and FLP in Baldari C, Murray JAH, Ghiara P, Cesareni G and Galeotti CL EMBO Journal (1987) 6: 229-234). The expression cassette and the genetic marker to select the transformants *(S. cerevisiae URA3* gene, Rose M, Grisafi P and Botsein D, Gene (1984) 29: 113-124) are flanked by the two sequences of the S. *cerevisiae* 25S rDNA gene which, as mentioned before, represent the regions able to recombine, by a double *crossing over,* with the homologous sequences in the *Hansenula* genome. In this construct, the human lysozyme cDNA was included as a *reporter* gene to verify the multicopy integration of the expression cassette into the *Hansenula* genome: actually, the lysozyme enzymatic (bacteriolytic) activity can be easily measured by quick methods on plates which make it easy to carry out the selection of the transformants.

The vector was prepared, in subsequent steps, in *Escherichia coli* DH5α strain, utilising the methods described for recombinant DNA in Sambrook J, Fritsch EF and Maniatis T: Molecular Cloning. A Laboratory Manual. 2nd Ed. Cold Spring Harbor Laboratory Press, Cold Spring Harbor, N.Y. 1989; therefore the vector contains outside the integration unit, a replication origin and an ampicillin resistance gene (not shown in Fig. 2).

The pRIMY-1 vector was prepared through a series of steps synthetically described hereinafter according to genetic engineering techniques reported for example in the manual cited above.

The *URA 3* gene (nt. 3-1166) was amplified from *Saccharomyces* cerevisiae (Accession number: K02207; Rose *et al.* 1984) by PCR, with the following primers:
URA3-for: ^{5'}GCGGGGATCCTTTTCAATTCAATTCATCAT^{3'}; and
URA3-rev: ^{5'}GAGGGATCCTCTAGAGCTTTTTCTTTCCAATTTT^{3'} adding a *Bam*HI site at both the ends of the gene and a *Xba*I site, more internally to *Bam*HI, at the 3' end; the so obtained amplified product (1186 base pairs) was digested with *Bam*HI and inserted in the compatible *Bcl*I-*Bgl*II sites of pScr25 plasmid (Rossolini et al., 1992), inside the DNA sequence coding for 25S RNA cloned as a 2.6 kb *Eco*RI fragment of the *Saccharomyces cerevisiae* 25S rDNA gene (nt. 1-2651 of the sequence J01355), obtaining the pScr25-URA23 plasmid.

The EcoRI fragment of this construct, containing the two rDNA *integration boxes* (i. e. the two non-contiguous segments of the 25S rDNA gene, the 0,55 kb EcoRI-*Bcl*I and 1.1 kb Bg*l*II-*Eco*RI, respectively, alleged in the sequence listing as Seq ID1 and Seq ID2, respectively), and internally the *URA3* gene, was then subcloned in the *Eco*RI site of pSP70 plasmid, (Promega, Madison, WI) (obtaining the pSP70-25S-URA3 plasmid).

*Hansenula polymorpha MOX* promoter was amplified by PCR from DL1 strain known in literature, utilising MOXP-for (^{5'}GGGAAGAACCGCGACATCTC^{3'}) and MOXP-rev primers, the latter introducing an additional *Sac*I site (^{5'}GGGAGCTCTTTGTTTTTGTACTTTAG^{3'}); the amplified product was cloned in the Smal site of the pBluescript SK vector polylinker (Stratagene, La Jolla, CA) to obtain the SK-PMOX plasmid. Its sequence was determined and is alleged in the sequence listing as Seq IDN3. The *Sac*I fragment of this construct was removed and inserted in the *Sac*I site of YIprD1-LYS plasmid (Rossolini *et al*., 1992), upstream the *Kluyveromyces lactis* killer toxin signal sequence, obtaining the YlprD1-MOX-LYS plasmid.

pRIMY-1 vector was finally obtained subcloning the 1.9 kb *Xba*I fragment of YIprD1-MOX-LYS plasmid (containing the MOX promoter plus the expression cassette composed of: *K. lactis* killer toxin signal sequence fused *in frame* with the cDNA coding for human lysozyme and transcriptional terminator derived from S. *cerevisiae* 2µm plasmid (Rossolini *et al.*, 1992) in the *Xba*I site of pSP70-25S-URA3 plasmid (Fig.1).

### Example 2. Transformation of H. polymorpha LR9 strain with pRIMY-1 vector and expression of the recombinant protein liysozyme.

### Growth and culture media used.

SD minimal medium : 0.67% (p/v) Yeast Nitrogen Base w/o amino acids (Difco) with 2% glucose (p/v) (SDD) or 1% methanol (SDM), and supplemented with uracile (50 µg/ml) when necessary. YP medium : 1% (p/v) yeast extract (Difco), 2% (p/v) peptone (Difco), with 2% glucose (YPD) or 1% methanol (YPM).

In some expression experiments, the carbon source was 2% glycerol or 1% methanol + 1% glycerol.

In experiments aimed to the measurement of the bacteriolytic activity expression level, comprising growth in culture broth for some days, 0.25% methanol was added daily.

Extraction of the integration unit from the vector was accomplished by digestion with *Cla*I enzyme. *Cla*I cuts at the ends of the *integration boxes* and generates a 4.7 kb fragment (see Fig. 2) which represents the integration unit, containing the rDNA *integration boxes* at the ends and, inside them, the expression cassette and URA3 gene. Extraction of the vector integration unit was carried out to facilitate the recombination at the level of the homologous regions on yeast genome. The integration unit permits the targeted integration of such unit into the chromosomal *locus* containing the *H. polymorpha* 25S rDNA genes.

About 5 total micrograms of pRIMY-1 vector were digested with *Cla*I enzyme and used to transform the *H. polymorpha* Ura⁻ LR9 strain (Roggenkamp R, Hansen H, Eckart M, Janowicz S and Hollenberg CP, Molecular and General Genetics (1986) 202: 302-308) according to the method described in Faber KN et al. J. Gen. Microbiol. (1992) 138: 2405-2416, which is reported herein: a "starter" culture of *H*. *polymorpha* yeast LR9 (Ura-) strain, was prepared inoculating a single colony in 10 ml of YPD medium and incubating overnight at 37°C under agitation at 300 r.p.m. Yeast cells were made competent by subsequent-dilution of the culture in 100 ml of fresh YPD medium until reaching an OD₆₀₀ of 0.1 and cultured in the same conditions until reaching, an OD₆₀₀ of 0.8. Cells were then collected, washed with 50 ml of solution A [1.0 M sorbitol, 10 mM bicine, pH 8.35, and 3% (v/v) ethylene glycol] and finally resuspended in 4 ml of solution A.

The so obtained *H. polymorpha* competent cells, in 0,2 ml aliquots, were directly used for the transformation, carried out with 5 µg of the linearized vector together with 40 µg of carrier DNA (fragmented and denatured DNA salmon sperm), in a total volume of 20 µl.

The mixture was placed at 37°C for 5 min., diluted with 1.5 ml of solution B [40% PEG4000, 200 mM bicine pH 8.35] and incubated at 30°C for 60 min. under mild agitation. The cells were then centrifuged for 5 min. at 3000 g, washed with 1.5 ml of solution C [150 mM NaCl, 10 mM bicine pH 8.35], again centrifuged and resuspended in 0.2 ml of solution C.

The transformation mixture, opportunely diluted, was then plated on SDD minimal medium and incubated for 3-4 days at 30°C.

After a 3-8 days growth period at 30°C *(Hansenula* transformants were initially cultured at a temperature less than optimal, equal to 37°C, to slow their growth and simplify the recombination and, therefore, exogenous DNA integration) 50 transformants were obtained, directly selected for their ability to grow on uracile-free minimal medium.

The transformants, i.e. the clones which became Ura⁺ because they had received the integration unit containing the *URA3* gene, were stable, i.e. capable of maintaining the Ura⁺ phenotype even after a 50 to 70 generations growth period in complete medium.

The obtained clones were initially analysed for the production of the heterologous protein (lysozyme), through growth on complete and minimal media, containing methanol or glucose as a carbon source and *Micrococcus luteus* cells as a substrate to detect the bacteriolytic activity (according to the assay called *lysoplate assay,* Castañón MJ, *et al.,* 1988). All the transformants were able to secrete active lysozyme, as demonstrated by the formation of a lysis ring around the colonies (see Figure 3).

Some of the transformants, selected among those producing the ring with the largest diameter in the plate assay, were analysed with the aim to quantify their lytic activity secreted in the culture medium: following a 4-day growth at 37°C in minimal or complete medium containing methanol as a carbon source, aliquots of the culture supernatants were analysed by *lysoplate assay* and the results are illustrated in Table 1. The amount of lysozyme produced resulted variable in the various analysed clones and related to the number of integrated copies for each clone (see Genetic Analysis, Example 3).

From the results obtained in these preliminary experiments, it was possible to observe that the amount of active lysozyme produced in this system is higher than previously obtained in *K. lactis* with an integrative vector and in S. *cerevisiae* with a replicative-type vector (Rossolini GM. et al. Gene, 1992, 119: 75-81). Further, analogous expression tests prepared growing the *Hansenula* recombinant clones in the presence of glycerol, or a glycerol/methanol mixture gave, in terms of lysozyme production, comparable results to those obtained growing the strains in the presence of the sole methanol, thus demonstrating the possibility to ferment the *H. polymorpha* transformants obtained according to the method and integrative vector of the invention, on simple carbon sources, with a one-step fermentation.

The yields of the analysed clones were further optimised (up to an order of magnitude) by large scale fermentation.

**Table 1. Human lysozyme secreted from the clones obtained through H. polymorpha LR9 transformation with the integrative pRIMY-1 vector.**

| Clones | Type of medium | |
|---|---|---|
| | YPM | SDM |
| | Amount of lysozyme (µg/ml) | |
| H1 | 2.5 | 2.1 |
| H3 | 4.2 | 2.5 |
| H4 | 12.2 | 10.0 |
| H7 | 4.7 | 10.7 |
| H11 | 5.0 | 4.5 |
| H20 | 4.3 | 5.3 |
| H23 | 5.0 | 5.0 |

Thus, these results indicate that the system set up according to the present invention allows to obtain production levels of the heterologous protein, higher than those obtained in the yeast recombinant system of the known art, growing the cells in minimal media and in the presence of inexpensive carbon sources such as methanol or glycerol. Such yields were further optimised by growing the transformants in a fermenter.

### Example 3. Genetic analysis of the transformant strains

The integration vector according to the invention is a substitutive-type vector *(replacement vector)* and contains an expression cassette and a selection marker cloned inside DNA segments capable of recombinating with the *Hansenula* chromosome, in the rDNA *locus:* the homologous annealing on both ends of the target chromosome (double *crossing over)* causes the integrated exogenous DNA to replace the chromosomal fragment, which serves as an integration *target.*

The analysis of the integrative events which took place after transformation was performed on 12 clones. For the analysis, the total DNA (about 2 µg) of each transformant, was digested with *Bam*HI enzyme which, as can be seen in Figure 2, cuts pRIMY-1 plasmid once, in correspondence of the integration unit (more precisely, *Bam*HI recognises a unique site which is located immediately upstream the *MOX* promoter, see fig. 2), and leaves, on the contrary, the *H. polymorpha* rDNA site intact: the digested DNA of the various clones was separated by agarose gel electrophoresis, transferred onto nitrocellulose and hybridized with appropriate probes. In Figure 4, the hybridization pattern of some of the 12 clones is illustrated, which was obtained using the *MOX* promoter as a probe. Having the position and the hybridization signal intensity, obtained with LR9 strain, as a reference point, it can be observed that all the transformants analysed show multiple tandem integrations: these can be identified as 4.7 kb positive bands, whose intensity is much higher than the single band present in LR9, representing the *Hansenula* genomic fragment with only one copy of *MOX.* The comparative analysis, conducted by a densitometer, of the intensities of such bands compared to that present in LR9, leaded to the quantification of the integration unit copy number present in every clone (see Figure 6).

To determine the distribution of the integration sites, the same samples (BamHl digestions) were hybridized with the human lysozyme cDNA (h-LYS probe) and with the S. *cerevisiae* 1.1 kb 25S rDNA fragment (rDNA probe). In Figure 5, the analysis carried out on 8 clones, selected as representative of the various integration situations, is depicted. Because the *Hansenula* rDNA *locus* does not contain *Bam*HI sites, the diffused hybridization signal (corresponding to the one with the highest molecular weight), comigrating with the non-digested genomic DNA and present in the hybridization with the rDNA probe in LR9 strain too, corresponds to integrative events in very distant rDNA units. In all cases, except for clones H13 and H17, a more intense 4.7 kb band is present, indicating a multiple tandem integration, in a head-to-tail orientation. Moreover in clones H17, H18 and H23 a 8.3 kb band is present, which can be presumably attributed to the multiple insertion of copies in a head-to-head orientation. On the other hand, clone H13 shows a single 6.1 kb hybridization signal when hybridized with h-LYS probe, and no discrete bands when hybridized with the 1.1 kb 25S rDNA fragment this suggests the presence of multiple tandem copies of the integration unit, and in a head-to-head orientation, which however misses the 1.1 kb *integration box* (see the scheme of the integrative events in Fig. 6). Furthermore in all the clones bands of minor intensity are present, whose heights correspond to more than 10 kb, which can be presumably interpreted by integrative (contiguous or alternate) events in different rDNA units. The number of integrated copies present in the control LR9 strain, calculated assuming the positiveness for the *MOX* probe signal as a reference for a single copy, varied between 8 (clone H1) and 45 (clone H5); furthermore, clones with the higher number of integrated copies contained the integration unit copies in a head-to-tail orientation. Finally analogous hybridization experiments but carried out on whole chromosomes of the various clones, further confirmed that in all the transformants the integrative events took place in the chromosome containing the rDNA genes and are therefore the result of site-specific integration events.

Consequently, the results presented here demonstrate that the integrative unit of the vector according to the invention is able to direct the stable and muticopy integration of an expression cassette containing a heterologous gene in different units of *H. polymorpha* rDNA.

Moreover the all transformants obtained produce, at different extents, high active lysozyme concentrations in the culture medium and this productivity is related to the number of the integrated copies in each clone, indicating that it is not necessary to analyse a high number of clones to obtain a producer strain and thus the integrative events, in multiple copies, directed by the vector according to the present invention take place with high probability.

## Claims

1. Plasmid vector for multicopy site-specific integration of an integration unit comprising an expression cassette into methotrophic yeast, wherein said expression cassette comprises the following functional regions:
a) a promoter active in methotrophic yeast
b) a heterologous DNA sequence coding for the protein, polypeptide or peptide of interest, the transcription of said heterologous DNA sequence being regulated by said promoter
c) transcription termination sequences
d) a selection marker selected from the group consisting of URA3, LEU2, HIS3, G418R
and wherein said expression cassette is flanked upstream (6') and downstream (3') by at least 50bp of the sequence coding for the S. cerevisiae 25S ribosomal RNA (GenBank access number J01355), **characterized in that** said promoter active in methiltrophic yeast is selected from the group consisting of: MOX, FMD, CAT-1 and DHAS promoter.

2. Plasmid vector according to Claim 1, wherein the MOX promoter is Seq IDN° 3 or its fragments of at least 600 nt.

3. Plasmid vector according to Claim 1, wherein said 258 ribosomal RNA coding sequences comprise Seq IDN1 and Seq IDN2.

4. Plasmid vector according to claim 1, further comprising a signal sequence for the secretion of heterologous protein.

5. Plasmid vector according to Claim 4, wherein the signal sequence is selected in the group consisting of: *K*. *Lactis* killer toxin signal sequence. *S*. *cerevisiae* killer toxin signal sequence, MFα1 factor signal sequence, *Aspergillus niger* glucoamylase signal sequence and *Schwannlomyces occidentalis* glucoamylase pre-pro-sequence.

6. Plasmid vector according to Claim 1, wherein said transcription termination sequence is the FLP fragment of the 2µm plasmid.

7. Plasmid vector pRIMY-1 for multicopy site-specific integration of heterologous DNA sequences in a metilotrophic yeast deposited at the CNMC collection (Collection Nationale de Cultures de Microorganismes, Institut Pasteur) on July 18th 2001, number 1-2706,

8. Integration unit for methotrophic yeasts comprising an expression cassette flanked upstream ('5) and downstream ('3) by DNA sequences coding for the yeast S. *cerevisiae* 25 S RNA (genbank access number: J01355) or by fragments of such sequence said fragments being at least 50 base pairs in lenght, and wherein said expression cassette comprises the following functional regions:
a) a promoter active In metilotrophic yeast selected from the group consisting of: MOX, FMD, CAT-1 and DHAS promoter
b) a heterologous DNA sequence coding for the protein, polypeptide or peptide of interest, the transcription of said heterologous DNA sequence being regulated by said promoter
c) transcription termination sequences
d) a selection marker selected from the group consisting of: URA3, LEU2, HIS3, G418R

9. Integration unit according to Claim 8 **characterised in that** said upstream (5') and downstream (3') sequences are Seq IDN1 e Seq IDN2 of the sequence listing.

10. Integration unit according to Claim 9 wherein said expression cassette comprises a selection marker.

11. Microorganism transformed with the vectors of claims 1-7 or with the integration unit according to Claims 8-10, **characterised** for being metilotrophic yeasts. 40

12. Microorganism according to Claim 11, wherein the metilotrophic yeast belongs to the Hansenula genus.

13. Microorganism according to Claim 12, wherein the metilitrophic, yeast is *Hansenula polymorpha.*

14. Process for integration of heterologous DNA sequences in the ribosomal *locus* coding for 25S RNA of metilotrophic yeasts, **characterised in that** the vectors of Claims 1-7 or the integration units according to Claims 8-10 are used.

15. Process according to Claim 14, wherein said Integration takes place by homologous recombination.

16. Process according to Claim 15, wherein the metilotrophic yeast belongs to *Pichia* and/or *Hansenula* genera.

17. Process according to Claim 16, wherein the yeast is *Hansenula polymorpha.*

18. Process for the production of recombinant heterologous proteins in a metilotrophic yeast, **characterised in that** the vectors according to Claims -1-7 or the integration units according to Claims 8-10 are used.

19. Process according to Claim 18, **characterized in that** such metilotrophic yeast is *Hansenula polymorpha.*

20. Process according to Claim 19, **characterised in that** it comprises the following steps:
- preparation of the yeast transformation-competent cells
- transformation of the *Hansenula polymorpha* strain with the vectors of Claims 1-7. optionally in the presence of a carrier DNA
- plating on selective medium and incubation of the transformed yeasts at a temperature comprised between 28°C and 32°C, for a time comprised between 60 is and 80 hours
- alternate growth on selective and non-selective media, at a temperature comprised between 28°C and 42°C
- phenotypic analysis of the transformants with best productivity features one-step fermentation of the selected transformant on simple carbon sources.

21. Process according to Claim 20, **characterised In that** the vectors of Claims 1-7 are digested by enzymatic digestion.

22. Kit for the expression of recombinant proteins in yeast comprising the Integration vector DNA according to Claims 1-8 and a yeast strain, preferably *H*. *Polymorpha,* optional In a lyophilised form and optionally comprising appropriate restriction enzymes.

## Patentansprüche

1. Plasmidvektor zur ortsspezifischen Multikopie-Integration einer Integrationseinheit mit einer Expressionskassette in eine methylotrophe Hefe, wobei die Expressionskassette die folgenden funktionellen Bereiche aufweist, nämlich:
a) einen in methylotropher Hefe aktiven Promotor,
b) eine heterologe DNA-Sequenz, die für das interessierende Protein, Polypeptid oder Peptid codiert, wobei die Transkription der heterologen DNA-Sequenz über den Promotor reguliert wird,
c) Sequenzen zur Termination der Transkription,
d) einen Selektionsmarker, der ausgewählt ist aus der Gruppe bestehend aus URA3, LEU2, HIS3, G418R,
wobei die Expressionskassette stromaufwärts (5') und stromabwärts (3') von zumindest 50 bp der Sequenz flankiert ist, die für die ribosomale 25S-RNA von S. *cerevisi*ae (GenBank-Zugriffsnummer J01355) codiert, **dadurch gekennzeichnet, dass** der in methylotropher Hefe aktive Promotor ausgewählt ist aus der Gruppe bestehend aus: MOX-, FMD-, CAT-1- und DHAS-Promotor.

2. Plasmidvektor gemäß Anspruch 1, wobei der MOX-Promotor der SEQ ID Nr. 3 oder Fragmenten hiervon mit zumindest 500 Nucleotiden entspricht.

3. Plasmidvektor gemäß Anspruch 1, wobei die Codierungssequenz für die ribosomale 25S-RNA die SEQ ID Nr. 1 und SEQ ID Nr. 2 aufweist.

4. Plasmidvektor gemäß Anspruch 1, der ferner eine Signalsequenz für die Sekretion von heterologem Protein aufweist.

5. Plasmidvektor gemäß Anspruch 4, wobei die Signalsequenz ausgewählt ist aus der Gruppe bestehend aus: Signalsequenz des Killertoxins von *K. Lactis,* Signalsequenz des Killertoxins von S. *cerevisiae,* Signalsequenz des MFα1-Faktors, Signalsequenz der Glucoamylase von *Aspergillus niger* und Pre-Pro-Sequenz der Glucoamylase von *Schwannlomyces occidentalis.*

6. Plasmidvektor gemäß Anspruch 1, wobei die Sequenz zur Termination der Transkription dem FLP-Fragment des 2 µm-Plasmides entspricht.

7. Plasmidvektor pRIMY-1 zur ortsspezifischen Multikopie-Integration von heterologen DNA-Sequenzen in eine methylotrophe Hefe, der am 18. Juli 2001 unter der Nummer 1-2705 bei der CNMC-Sammlung (Collection Nationale de Cultures de Microorganismes, Institut Pasteur) hinterlegt wurde.

8. Integrationseinheit für methylotrophe Hefen, die eine Expressionskassette aufweist, die stromaufwärts (5') und stromabwärts (3') von DNA-Sequenzen flankiert ist, die für die 25S-RNA der Hefe S. *cerevisiae* (Genbank-Zugriffsnummer J01355) codieren, oder von Fragmenten einer solchen Sequenz, wobei die Fragmente eine Länge von zumindest 50 Basenpaaren haben, wobei die Expressionskassette die folgenden funktionellen Bereiche aufweist, nämlich:
a) einen in methylotropher Hefe aktiven Promotor, der ausgewählt ist aus der Gruppe bestehend aus: MOX-, FMD-, CAT-1- und DHAS-Promotor,
b) eine heterologe DNA-Sequenz, die für das interessierende Protein, Polypeptid oder Peptid codiert, wobei die Transkription der heterologen DNA-Sequenz über den Promotor reguliert wird,
c) Sequenzen zur Termination der Transkription,
d) einen Selektionsmarker, der ausgewählt ist aus der Gruppe bestehend aus: URA3, LEU2, HIS3, G418R.

9. Integrationseinheit gemäß Anspruch 8, **dadurch gekennzeichnet, dass** die stromaufwärts (5') und stromabwärts (3') gelegenen Sequenzen den SEQ ID Nr. 1 und SEQ ID Nr. 2 des Sequenzprotokolls entsprechen.

10. Integrationseinheit gemäß Anspruch 9, wobei die Expressionskassette einen Selektionsmarker aufweist.

11. Mikroorganismus, der mit den Vektoren der Ansprüche 1 bis 7 oder mit der Integrationseinheit gemäß der Ansprüche 8 bis 10 transformiert ist, **dadurch gekennzeichnet, dass** es sich um methylotrophe Hefen handelt.

12. Mikroorganismus gemäß Anspruch 11, wobei die methylotrophe Hefe zu der Gattung *Hansenula* gehört.

13. Mikroorganismus gemäß Anspruch 12, wobei die methylotrophe Hefe *Hansenula polymorpha* entspricht.

14. Verfahren zur Integration von heterologen DNA-Sequenzen in den ribosomalen Locus, der für die 25S-RNA von methylotrophen Hefen codiert, **dadurch gekennzeichnet, dass** die Vektoren der Ansprüche 1 bis 7 oder die Integrationseinheiten gemäß der Ansprüche 8 bis 10 verwendet werden.

15. Verfahren gemäß Anspruch 14, wobei die Integration durch homologe Rekombination erfolgt.

16. Verfahren gemäß Anspruch 15, wobei die methylotrophe Hefe zu der Gattung *Pichia* und/oder *Hansenula* gehört.

17. Verfahren gemäß Anspruch 16, wobei die Hefe *Hansenula polymorpha* entspricht.

18. Verfahren zur Herstellung von rekombinanten heterologen Proteinen in einer methylotrophen Hefe, **dadurch gekennzeichnet, dass** die Vektoren gemäß der Ansprüche 1 bis 7 oder die Integrationseinheit gemäß der Ansprüche 8 bis 10 verwendet werden.

19. Verfahren gemäß Anspruch 18, **dadurch gekennzeichnet, dass** die methylotrophe Hefe *Hansenula polymorpha* entspricht.

20. Verfahren gemäß Anspruch 19, **dadurch gekennzeichnet, dass** dieser die folgenden Schritte aufweist, nämlich:
- Herstellung der transformationskompetenten Hefezellen,
- Transformation des Stammes *Hansenula polymorpha* mit den Vektoren der Ansprüche 1 bis 7, ggf. in Gegenwart einer Träger-DNA,
- Ausplattieren auf Selektionsmedium und Inkubation der transformierten Hefen bei einer Temperatur, die bei 28°C bis 32°C liegt, für eine Zeit, die bei 60 bis 80 Stunden liegt,
- abwechselndes Wachstum auf Selektions- und Nicht-Selektionsmedium bei einer Temperatur, die bei 28°C bis 42°C liegt,
- phänotypische Analyse der Transformanten mit den besten Produktivitätseigenschaften,
- Ein-Schritt-Fermentierung der selektierten Transformante auf einfachen Kohlenstoffquellen.

21. Verfahren gemäß Anspruch 20, **dadurch gekennzeichnet, dass** die Vektoren der Ansprüche 1 bis 7 durch enzymatischen Verdau verdaut werden.

22. Kit zur Expression von rekombinanten Proteinen in Hefe, das die Integrationsvektor-DNA gemäß der Ansprüche 1 bis 8 und einen Hefestamm, vorzugsweise *H. Polymorpha,* ggf. in lyophilisierter Form aufweist, und ggf. geeignete Restriktionsenzyme aufweist.

## Revendications

1. Vecteur plasmide pour l'intégration multicopie dirigée, dans une levure méthylotrophique, d'une unité d'intégration comprenant une cassette d'expression, dans lequel ladite cassette d'expression comprend les régions fonctionnelles suivantes :
(a) un promoteur actif dans la levure méthylotrophique
(b) une séquence d'ADN hétérologue codant pour la protéine, le polypeptide ou le peptide d'intérêt, la transcription de ladite séquence d'ADN hétérologue étant régulée par ledit promoteur,
(c) des séquences de terminaison de la transcription
(d) un marqueur de sélection choisi dans le groupe se composant de URA3, LEU2, HIS3 et G418R
et dans lequel ladite cassette d'expression est flanquée en amont (5') et en aval (3') par au moins 50 pb (paires de base) de la séquence codant pour l'ARN ribosomal 25S de la *S. cerevisiae* (GenBank No. Accès J01355), ***caractérisé en ce que*** ledit promoteur actif dans la levure méthylotrophique est choisi dans le groupe se composant des promoteurs MOX, FMD, CAT-1 et DHAS.

2. Vecteur plasmide selon la revendication 1, dans lequel le promoteur MOX est la Seq ID NO. 3 ou ses fragments d'au moins 500 nt.

3. Vecteur plasmide selon la revendication 1, dans lequel lesdites séquences codant pour l'ARN ribosomal 25S comprennent la Seq ID NO. 1 et la Seq ID NO. 2.

4. Vecteur plasmide selon la revendication 1, comprenant de plus une séquence signal pour la sécrétion de la protéine hétérologue.

5. Vecteur plasmide selon la revendication 4, dans lequel la séquence signal est choisie dans le groupe se composant de : la séquence signal de la toxine létale de *K. Lactis,* la séquence signal de la toxine létale de S. *cerevisiae,* de la séquence signal du facteur MFα1, de la séquence signal de la glucoamylase *d'Aspergillus niger* et de la pré-pro-séquence de la glucoamylase de *Schwanniomyces occidentalis.*

6. Vecteur plasmide selon la revendication 1, dans lequel ladite séquence de terminaison de transcription est le fragment FLP du plasmide 2*µ*m.

7. Vecteur plasmide pRIMY-1 pour l'intégration multicopie dirigée de séquences d'ADN hétérologue dans une levure méthylotrophique déposée à la collection CNCM (Collection Nationale de Cultures de Microorganismes, Institut Pasteur) le 18 Juillet 2001, numéro 1-2705.

8. Unité d'intégration pour des levures méthylotrophiques comprenant une cassette d'expression flanquée en amont (5') et en aval (3') par des séquences d'ADN codant pour l'ARN ribosomal 25S de la *S. cerevisiae* (GenBank No. J01355) ou par des fragments de ces séquences, lesdits fragments ayant une longueur d'au moins 50 paires de base, et dans laquelle ladite cassette d'expression comprend les régions fonctionnelles suivantes :
(a) un promoteur actif dans la levure méthylotrophique choisi dans le groupe se composant des promoteurs MOX, FMD, CAT-1 et DHAS.
(b) une séquence d'ADN hétérologue codant pour la protéine, le polypeptide ou le peptide d'intérêt, la transcription de ladite séquence d'ADN hétérologue étant régulée par ledit promoteur,
(c) des séquences de terminaison de la transcription
(d) un marqueur de sélection choisi dans le groupe se composant de URA3, LEU2, HIS3 et G418R.

9. Unité d'intégration selon la revendication 8, ***caractérisée en ce que*** lesdites séquences en amont (5') et en aval (3') sont la Seq ID NO. 1 et la Seq ID NO. 2 de la liste des séquences.

10. Unité d'intégration selon la revendication 9, dans laquelle ladite cassette d'expression comprend un marqueur de sélection.

11. Microorganisme transformé avec les vecteurs des revendications 1 à 7, ou avec l'unité d'intégration selon les revendications 8 à 10, ***caractérisé*** comme étant des levures méthylotrophiques.

12. Microorganisme selon la Revendication 11, dans lequel la levure méthylotrophique appartient au genre *Hansenula.*

13. Microorganisme selon la Revendication 12, dans lequel la levure méthylotrophique est *Hansenula polymorpha.*

14. Procédé d'intégration de séquences d'ADN hétérologue dans le *locus* ribosomal codant pour l'ARN 25S de levures méthylotrophiques, ***caractérisé en ce que*** les vecteurs des revendications 1 à 7 ou les unités d'intégration selon les revendications 8 à 10 sont utilisés.

15. Procédé selon la revendication 14, dans lequel ladite intégration se fait par recombinaison homologue.

16. Procédé selon la revendication 15, dans lequel la levure méthylotrophique appartient au genre *Pichia* et/ou *Hansenula.*

17. Procédé selon la revendication 16, dans lequel la levure est *Hansenula polymorpha.*

18. Procédé pour la production de protéines hétérologues recombinantes dans une levure méthylotrophique, ***caractérisé en ce que*** les vecteurs selon les revendications 1 à 7 ou les unités d'intégration selon les revendications 8 à 10 sont utilisés.

19. Procédé selon la revendication 18, ***caractérisé en ce que*** cette levure méthylotrophique est *Hansenula polymorpha.*

20. Procédé selon la revendication 19, ***caractérisé en ce qu'**il* comprend les étapes suivantes ;
- préparation des cellules compétentes pour la transformation de la levure
- transformation de la souche *Hansenula polymorpha* avec les vecteurs des revendications 1 à 7, facultativement en présence d'un ADN porteur
- plaquage sur un milieu sélectif et incubation des levures transformées à une température comprise entre 28°C et 32°C, pour une durée comprise entre 60 et 80 heures,
- croissance alternée sur des milieux sélectifs et non sélectifs, à une température comprise entre 28°C et 42°C
- analyse phénotypique des transformants avec les meilleures caractéristiques de productivité
- fermentation en une étape du transformant sélectionné sur des sources de carbone simples.

21. Procédé selon la revendication 20, ***caractérisé en ce que*** les vecteurs des revendications 1 à 7 sont digérés par digestion enzymatique.

22. Trousse pour l'expression de protéines recombinantes dans une levure, comprenant l'ADN du vecteur d'intégration selon les revendications 1 à 8 et une souche de levure, de manière préférée *H. Polymorpha,* facultativement sous forme lyophilisée et comprenant facultativement des enzymes de restriction appropriées.


### SEQUENCE LISTING

<110> BIOANALISI CENTRO SUD di Perseo Sinibaldo S.n.c.
<120> Vector for site specific integration of heterologous DNA sequences into metilotrophic yeast integration vector yeast
<130> 2606PTWO
<140> PCT/IB02/03086
   <141> 2002-08-05
<150>. M120OIA001728
   <151> 2001-08-06
<160> 3
<170> PatentIn Ver. 2.0
<210> 1
   <211> 548
   <212> DNA
   <213> Saccharomyces cerevisiae
<220>
   <223> "integration box" for the ribosomal locus 25S
<400> 1
   aattccgtga tgggccttta ggttttacca actgcggcta atcttttttt atactgagcg 60
   tattggaacg ttatcgataa gaagagagcg tctaggcgaa caatgttctt aaagtttgac 120
   ctcaaatcag gtaggagtac ccgctgaact taagcatatc aataagcgga ggaaaagaaa 180
   ccaaccggat tgccttagta acggcgagtg aagcggcaaa agctcaaatt tgaaatctgg 240
   taccttcggt gcccgagttg taatttggag agggcaactt tggggccgtt ccttgtctat 300
   gttccttgga acaggacgtc atagagggtg agcatcccgt gtggcgagga gtgcggttct 360
   ttgtaaagtg ccttcgaaga gtcgagttgt ttgggaatgc agctctaagt gggtggtaaa 420
   ttccatctaa agctaaatat tggcgagaga ccgatagcga acaagtacag tgatggaaag 480
   atgaaaagaa ctttgaaaag agagtgaaaa agtacgtgaa attgttgaaa gggaagggca 540
   tttgatca                                                          548
<210> 2
   <211> 960
   <212> DNA
   <213> Saccharomyces cerevisiae
<220>
   <223> "integration box" for the ribosomal locus 25S.
<400> 2
   agatcttggt ggtagtagca aatattcaaa tgagaacttt gaagactgaa gtggggaaag 60
   gttccacgtc aacagcagtt ggacgtgggt tagtcgatcc taagagatgg ggaagctccg 120
   tttcaaaggc ctgattttat gcaggccacc atcgaaaggg aatccggtaa gattccggaa 180
   cttggatatg gattcttcac ggtaacgtaa ctgaatgtgg agacgtcggc gcgagccctg 240
   ggaggagtta tcttttcttc ttaacagctt atcaccccgg aattggttta tccggagatg 300
   gggtcttatg gctggaagag gccagcacct ttgctggctc cggtgcgctt gtgacggccc 360
   gtgaaaatcc acaggaagga atagttttca tgctaggtcg tactgataac cgcagcaggt 420
   ctccaaggtg aacagcctct agttgataga ataatgtaga taagggaagt cggcaaaata 480
   gatccgtaac ttcgggataa ggattggctc taagggtcgg gtagtgaggg ccttggtcag 540
   acgcagcggg cgtgcttgtg gactgcttgg tggggcttgc tctgctaggc ggactacttg 600
   cgtgccttgt tgtagacggc cttggtaggt ctcttgtaga ccgtcgcttg ctacaattaa 660
   cagatcaact tagaactggt acggacaagg ggaatctgac tgtctaatta aaacatagca 720
   ttgcgatggt cagaaagtga tgttgacgca atgtgatttc tgcccagtgc tctgaatgtc 780
   aaagtgaaga aattcaacca agcgcgagta aacggcggga gtaactatga ctctcttaag 840
   gtagccaaat gcctcgtcat ctaattagtg acgcgcatga atggattaac gagattccca 900
   ctgtccctat ctactatcta gcgaaaccac agccaaggga acgggcttgg cagaatcagc 960
<210> 3
   <211> 985
   <212> DNA
   <213> Hansenula polymorpha
<220>
   <221> promoter
   <222> (1)..(985)
   <223> methanol oxidase promoter
<400> 3
   ggaagaaccg cgacatctcc aacagctggg ccgtgttgag gatgagccgg acgtcgttga 60
   acgagggcgc cacaggccgg cgtttgctga tggcgcggcg ctcgtcctcg atgtagaagg 120
   ccttttccag aggcagtctg gtgaaaaagt tgccaacgct cggaaccagc tgcacgagcc 180
   gagacaattc gggggtgccg gctttggtca tttcaatgtt gtcgtcgatg aggagttcga 240
   ggtcgtggaa gatttctgcg taacggcgtt ttgcctcaga gttcaccatg agatcgtcca 300
   cggcggagat gccgttgctc ttcaccgcgt acaggacgaa cggcgtggcc aacaggccct 360
   ttatccactc tatgaggccg tctcgacggt gttccttgag tgcatactcc actctgtagc 420
   gactagtcat ccggaggctg ggctttctgc gctgggtgta ctaattaatt gttgccgcac 480
   ctgtacgggg taccttgcat ccttgcaccg caactgaaat aaacccactc gctttagccg 540
   tcgcgtaaaa ctcgtgaatc tggcaactga gggggttctg cagccgcaac caaactttat 600
   cgctttgagg acgcagctgg atggtgtcat gtgaggctct gttctctggc gtagcctaca 660
   acgtgacttt gcctaagcgg acggccctac ccttagctgc ctgcgcctgc taccagaaaa 720
   tcactagaac agcagagggc cgatgtggta attggtgcgg tgtcggccag tctgtttctc 780
   cacagtgcaa atgcgggtga accggccaga aagcaaattt cttatgctac cgtgcagtga 840
   ctccgacatc cccagttttt gccctacttg atcacagatg gggtcagcac tgtcgctaag 900
   tgcacccagt cgtccccaca cgcgcaatct ataaatactg ccgccagtgc acggtggtga 960
   catcaatcta aagtacaaaa acaaa                                        985